# EUROPEAN PATENT SPECIFICATION

(11) **EP 1 875 940 B1**
(45) Date of publication and mention of the grant of the patent: **21.10.2009**
(21) Application number: 07018178.9
(22) Date of filing: 01.07.2004
(51) Int. Cl.: A61M 25/06

(54) **Intravenous catheter introducing device**
Intravenöse Kathetereinführvorrichtung
Dispositif d'introduction intraveineuse d'un cathéter

(43) Date of publication of application: 09.01.2008
(62) Divisional of application: 04253967.6
(73) Proprietor: Shue, Ming-Jeng, Hsi District, Taichung City (TW); Huang, Deborah, Taichung City (TW); Shue, Philip, Hsi District Taichung City (TW)
(72) Inventor: Shue, Ming-Jeng, Hsi District, Taichung City (TW); Huang, Deborah, Taichung City (TW); Shue, Philip, Hsi District Taichung City (TW)
(74) Representative: Green, Mark Charles

(56) References cited:
- WO-A-02/41932
- WO-A-97/05912
- US-A- 5 575 773
- US-B1- 6 325 781
- US-B1- 6 547 762

## Description

This invention relates to an intravenous catheter introducing device, more particularly to an intravenous catheter introducing device with a needle cannula which is retractable into a barrel for safe disposal.

Referring to Figs. 1 and 2, a self-retracting IV catheter 7introducer disclosed in U.S. patent No. 5,989,220 is shown to include a catheter connector assembly including a barrel 10 enclosing a retraction body 11 which has a front portion 111 that carries a needle cannula 12 and that extends through an opening in front of the barrel 10, and a rear portion 112 that is mounted within the barrel 10. The front portion 111 has a connection surface 111a which is frictionally engaged with a corresponding connection surface 131 of a catheter hub 13 so as to prevent rearward retraction of the retraction body 11. A biasing spring 15 is compressed against a ledge 101 at the front of the barrel 10, and a spring seat 113 of the retraction body 11. In use, the needle cannula 12 which extends through a flexible catheter 14 is inserted into a patient's vein to introduce the catheter 14 into the vein for intravenous delivery of fluid. The catheter hub 13 is then separated from the retraction body 11 by forcibly pulling the barrel 10 while holding the catheter hub 13 so that the catheter hub 13 is separated from the front portion 111 of the retraction body 11 by loosening of the connection surface 111a, and the retraction body 11 is immediately and automatically forced into the barrel 10 by the biasing spring 15, thereby drawing the used needle cannula 12 behind it. However, since the needle cannula 12 is drawn as soon as the catheter hub 13 is disengaged from the retraction body 11, the drawing operation is not manually controllable, which may lead to an undesirable withdrawal of the needle cannula 12 that may result in an accident, such as dropping of the barrel 10.

The object of the present invention is to provide an intravenous catheter introducing device which can be operated easily and safely to retract a used needle cannula with one hand.

WO 97/05912 discloses the features of the preamble of claim 1.

According to this invention, an intravenous catheter is provided as defined in claim 1.

Other features and advantages of the present invention will become apparent in the following detailed description of the preferred embodiments of the invention, with reference to the accompanying drawings, in which:

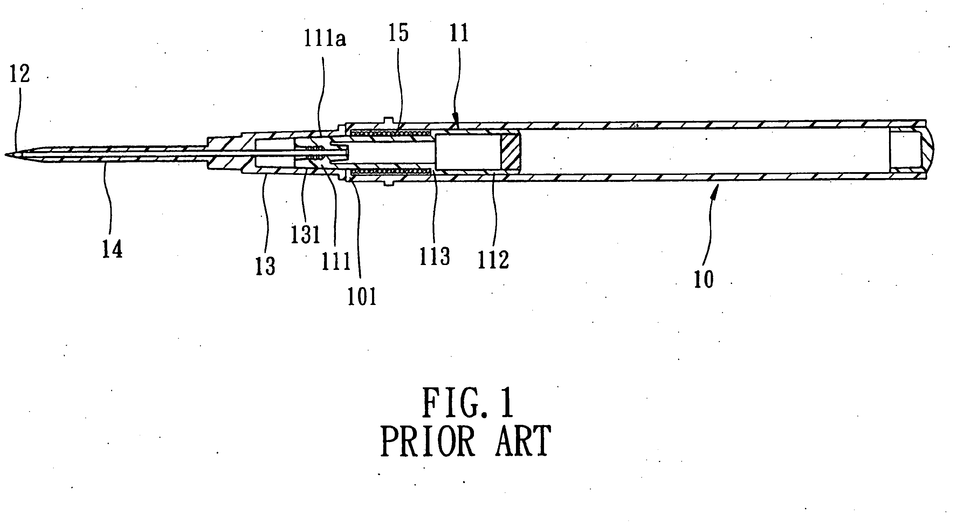

Fig. 1 is a sectional view of a conventional IV catheter introducer in a ready-to-use position;

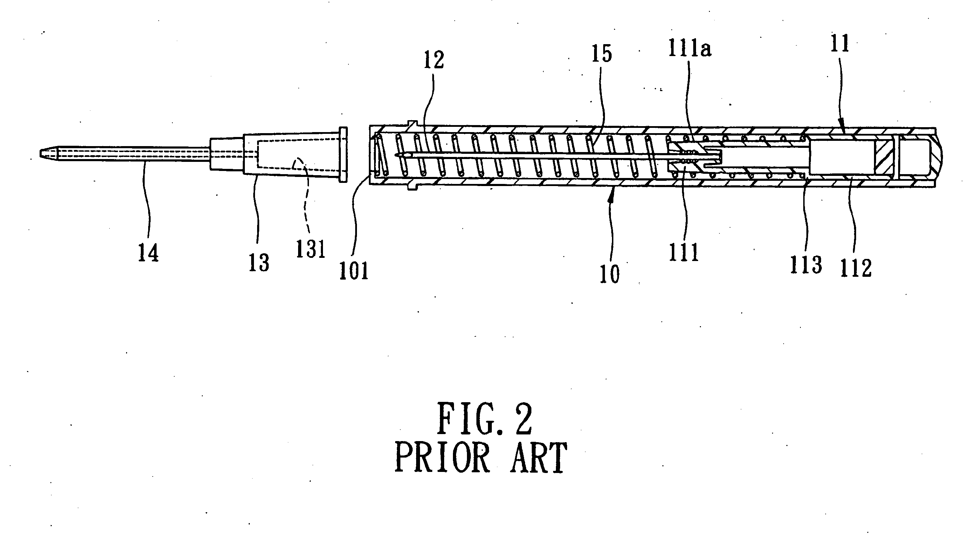

Fig. 2 is a sectional view of the conventional IV catheter introducer in a retracted position;
Fig. 3 is an exploded sectional view of a first example of an intravenous catheter introducing device not falling within the scope of the invention.
Fig. 4 is a sectional view of the first example in a ready-to-use position;
Fig. 5 is a sectional view of the first example in a retracted position;
Fig. 6 is a sectional view of a second example of an intravenous catheter introducing device not falling within the scope of the invention in a ready-to-use position;
Fig. 7 is a sectional view of the second example in a retracted position;
Fig. 8 is a sectional view of a third example of an intravenous catheter introducing device not falling within the scope of the invention in a ready-to-use position;
Fig. 9 is a sectional view of the third example in a retracted position;
Fig. 10 is a sectional view of a fourth example of an intravenous catheter introducing device not falling within the scope of the invention in a ready-to-use position;
Fig. 11 is a sectional view of the fourth example in a retracted position;
Fig. 12 is a fragmentary exploded sectional view of a first preferred embodiment of an intravenous catheter introducing device according to this invention; and
Fig. 13 is a sectional view of a second preferred embodiment of an intravenous catheter introducing device according to this invention in a ready-to-use position.

Before the present invention is described in greater detail, it should be noted that same reference numerals have been used to denote like elements throughout the specification.

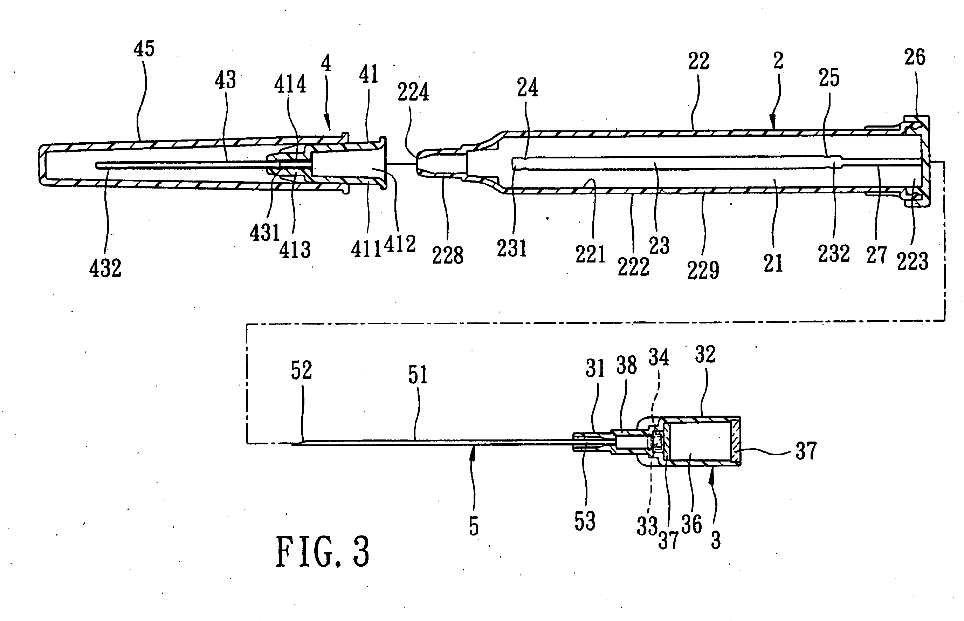

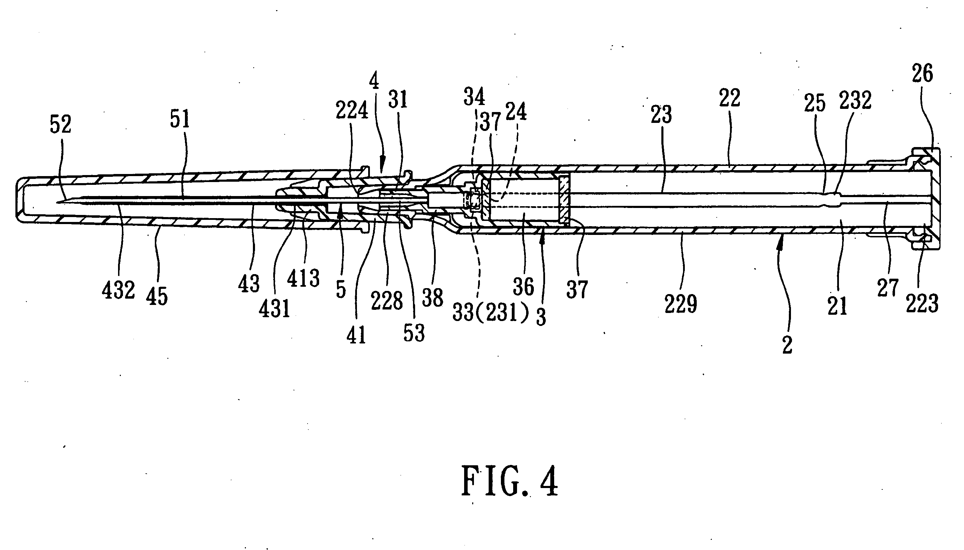

Referring to Figs. 3 and 4, the example of an intravenous catheter introducing device according to the present invention is shown to comprise a barrel 2, a needle hub 3, a needle cannula 5, and a catheter connection assembly 4.

The barrel 2 has front and rear open ends 224,223 opposite to each other in a longitudinal direction, and a surrounding barrel wall 22 which interconnects and which is interposed between the front and rear open ends 224,223. The surrounding barrel wall 22 includes a front smaller-diameter wall portion 228 and a rear larger-diameter wall portion 229 which are opposite to each other in the longitudinal direction and which are proximate to the front and rear open ends 224,223, respectively. The surrounding barrel wall 22 has an inner barrel wall surface 221 which surrounds an axis in the longitudinal direction and which confines a passage 21 communicated with the front and rear open ends 224,223, and an outer barrel wall surface 222 opposite to the inner barrel wall surface 221 in radial directions relative to the axis.

A releasably retaining member includes a retaining hole 231 which is formed in the outer barrel wall surface 222 of the larger-diameter wall portion 229, and which extends in a radial direction through the inner barrel wall surface 221. The rear larger-diameter wall portion 229 has an elongated guideway 23 which extends from the outer barrel wall surface 222 through the inner barrel wall surface 221 in the radial direction, and which is elongated from the retaining hole 231 rearwardly and in the longitudinal direction to terminate at a rear retaining end 232. The elongated guideway 23 has front and rear constricted regions 24,25 which are formed immediately behind the retaining hole 231 and immediately in front of the rear retaining end 232, respectively.

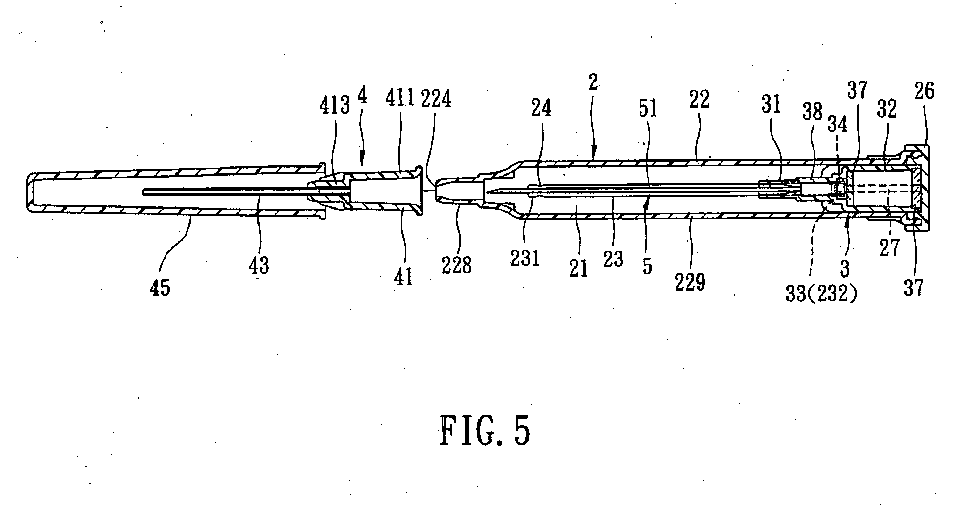

The needle cannula 5 has a front segment 51 terminating at a tip end 52, and a rear connecting end 53 opposite to the front segment 51 along the axis. The needle hub 3 is inserted into the passage 21 from the rear open end 223, which is closed by a closure cap 26, and is slidable relative to the surrounding barrel wall 22 along the axis between front and rear positions to be proximate to the front open end 224 and the rear open end 223, respectively. The needle hub 3 includes a front holding portion 31 which holds the rear connecting end 53 of the needle cannula 5 such that when the needle hub 3 is in the front position, the needle cannula 5 is placed in a position of use, as shown in Fig. 4, where the front segment 51 extends forwardly of the front open end 224 for ready use, and when the needle hub 3 is in the rear position, the needle cannula 5 is placed in a disposal position, as shown in Fig. 5, where the front segment 51 retreats into the passage 21. The needle hub 3 further includes a rear shell portion 32 which is disposed opposite to the front holding portion 31 along the axis and which is received in the passage 21 at the larger-diameter wall portion 229. The rear shell portion 32 surrounds the axis and defines a flashback chamber 36 which is fluidly communicated with the needle cannula 5. Two air-permeable members 37 are in engagement with the rear shell portion 32 to enclose the flashback chamber 36, and are made from a porous filter material for passage of air displaced by the fluid so as to restrain the possible fast flashback blood flow. In addition, the needle hub 3 has an intermediate portion 38 which interconnects the front holding portion 31 and the rear shell portion 32 to communicate the needle cannula 5 with the flashback chamber 36 and which is light transmissible to permit viewing of blood flowing therethrough.

The releasably retaining member further includes an engaging peg 33 disposed on and extending in the radial direction from the rear shell portion 32 to terminate at a shifted end which extends radially and outwardly of the outer barrel wall surface 222. The engaging peg 33 is slidable along the elongated guideway 23 from the retaining hole 231 to the rear retaining end 232 when the needle hub 3 slides from the front position to the rear position. Thus, the engaging peg 33 is engageable in the retaining hole 231 or the rear retaining end 232 to form an interengagement between the larger-diameter wall portion 229 and the rear shell portion 32. When the needle hub 3 is disposed at the front or rear position, axial movement of the needle hub 3 relative to the barrel 2 is arrested by a corresponding one of the front and rear constricted regions 24,25. Once the engaging peg 33 is forced through one of the front and rear constricted regions 24,25, movement of the engaging peg 33 is arrested by virtue of a snap-fit in a corresponding one of the retaining hole 231 and the rear retaining end 232 so as to position the needle hub 3 in a corresponding one of the front and rear positions. The larger-diameter wall portion 229 further has a split 27 which extends from the rear retaining end 232 to the rear open end 223 so as to vest the elongated guideway 23 with an increased flexibility along the radial direction, thereby facilitating the forced movement of the engaging peg 33 through the front and rear constricted regions 24,25, and facilitating the insertion of the engaging peg 33 into the elongate guideway 23 through the split 27.

An enlarged actuator 34 is formed integrally with the shifted end of the engaging peg 33, and is disposed outwardly of and is slidable relative to the outer barrel wall surface 222 so as to be disengaged from the retaining hole 231, thereby permitting the axial movement of the needle hub 3 to the rear position along the elongated guideway 23.

The catheter connection assembly 4 includes a catheter hub 41, a flexible tubular catheter 43, and a tip protector 45.

The catheter hub 41 includes a sleeve portion 411 which is detachably sleeved on the smaller-diameter wall portion 228 of the barrel 2 and which defines a duct 412 along the axis, and a tip portion 413 which is opposite to the sleeve portion 411 along the axis, and which defines a through hole 414 that communicates with the duct 412 along the axis, and that permits extension of the front segment 51 of the needle cannula 5 therethrough.

The tubular catheter 43 has a proximate segment 431 which is inserted into the through hole 414 and which extends along the axis to be fluidly communicated with the duct 412, and a distal segment 432 which extends from the proximate segment 431 along the axis to be disposed forwardly of the tip portion 413 of the catheter hub 41 so as to surround and sheathe the front segment 51 of the needle cannula 5 while permitting the tip end 52 to project forwardly of the distal segment 432 when the needle cannula 5 is placed in the position of use.

In use, after the tip protector 45 is removed, the tip end 52 of the needle cannula 5 is inserted into the patient's vein so as to introduce the tubular catheter 43 into the vein. Blood flowing into the flashback chamber 36 is visible from the intermediate portion 38 of the needle hub 3 so that the user can check whether the needle cannula 5 has been inserted properly into the vein. Referring to Fig. 5, the user can then separate the catheter hub 41 from the barrel 2 by holding the catheter hub 41 with one hand and holding and pulling the surrounding barrel wall 22 with the other hand. At the same time, the actuator 34 is operated with a finger of the hand holding the surrounding barrel wall 22 to move the engaging peg 33 rearwardly along the elongated guideway 23 so as to bring the needle hub 3 to the rear position, thereby placing the needle cannula 5 in the disposal position, where the front segment 51 retreats inwardly and rearwardly of the front open end 224 for safe disposal.

As illustrated, during operation, the user can hold the barrel 2 with one hand and operate the actuator 34 with a finger of the hand to cause the needle hub 3 to move to the rear position for drawing the used needle cannula 5 into the passage 21. Therefore, the operation is controllable by the user and is convenient to conduct. Besides, undesirable accidents can be avoided.

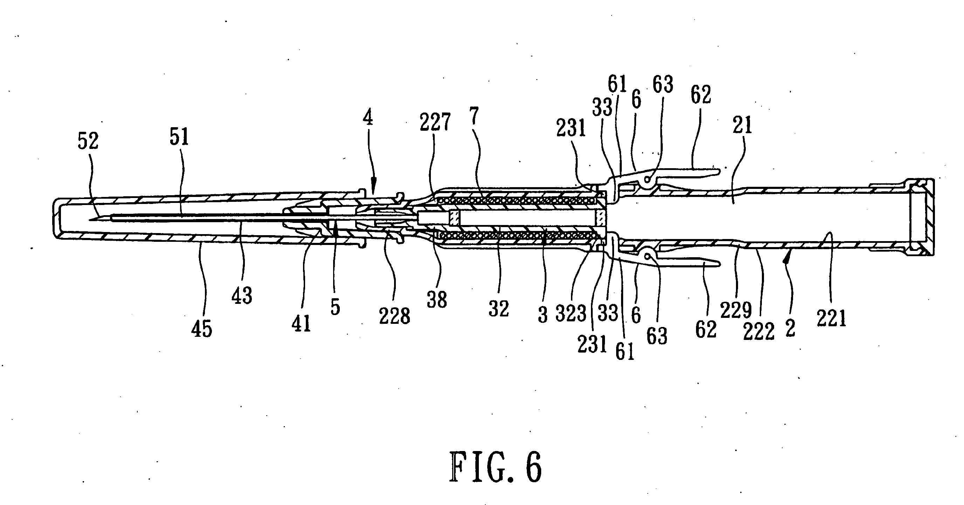

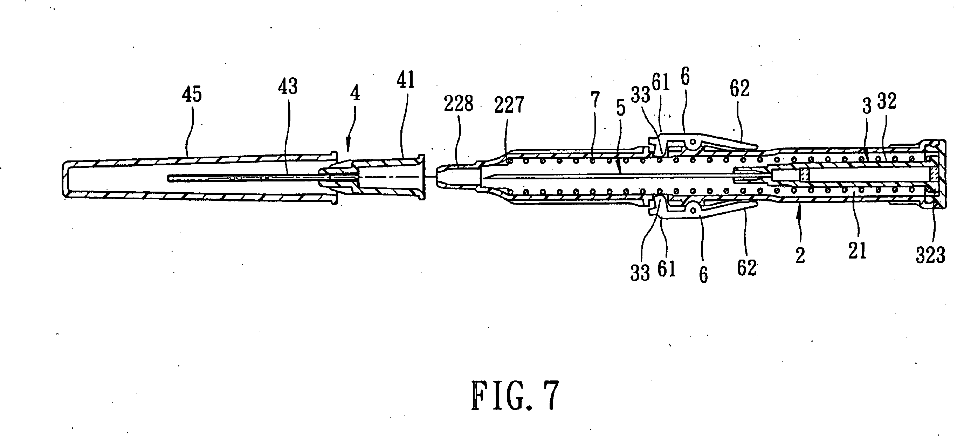

Referring to Figs. 6 and 7, the second example of an intravenous catheter introducing device is shown to be similar to the first example in construction. The differences reside in that the releasably retaining member includes two retaining holes 231 formed in the larger-diameter wall portion 229, and that the actuator includes two triggering members 6 formed as levers. Each of the triggering members 6 is mounted pivotally on the outer barrel wall surface 222 at a fulcrum point 63, and includes a weight end 61 formed integrally with the engaging peg 33, and a power end 62 disposed at an opposite side of the weight end 61 relative to the fulcrum point 63 so as to be actuated to move the engaging peg 33 in the radial direction to withdraw the engaging peg 33 from the passage 21 in the barrel 2 to thereby release the needle hub 3.

Furthermore, the inner barrel wall surface 221 of the larger-diameter wall portion 229 has a shoulder 227 with ribs formed adjacent to the smaller-diameter wall portion 228. The rear shell portion 32 of the needle hub 3 has an annular rear flange 323 confronting the shoulder 227 in the longitudinal direction so as to define a biasing member receiving space therebetween and outside of the rear shell portion 32. A biasing member, such as a coiled spring 7, is received in the biasing member receiving space, and includes front and rear spring ends abutting against the ribs of the shoulder 227 and the flange 323, respectively, such that the coiled spring 7 is compressed by the needle hub 3 when the needle hub 3 is in the front position. Due to the provision of the coiled spring 7, when the power ends 62 of the triggering members 6 are depressed at the same time with the fingers to smoothly retract the engaging pegs 33 radially, the needle hub 3 is moved to the rear position so as to bring the needle cannula 5 to the disposal position, thereby preventing shaking of the needle cannula 5 during the retraction of the needle cannula 5 to help lessen the patient' s pain.

It is noted that although the actuator in this example is exemplified as including two triggering members 6, one triggering member 6 will be sufficient to perform the function of retaining and releasing the needle hub 3.

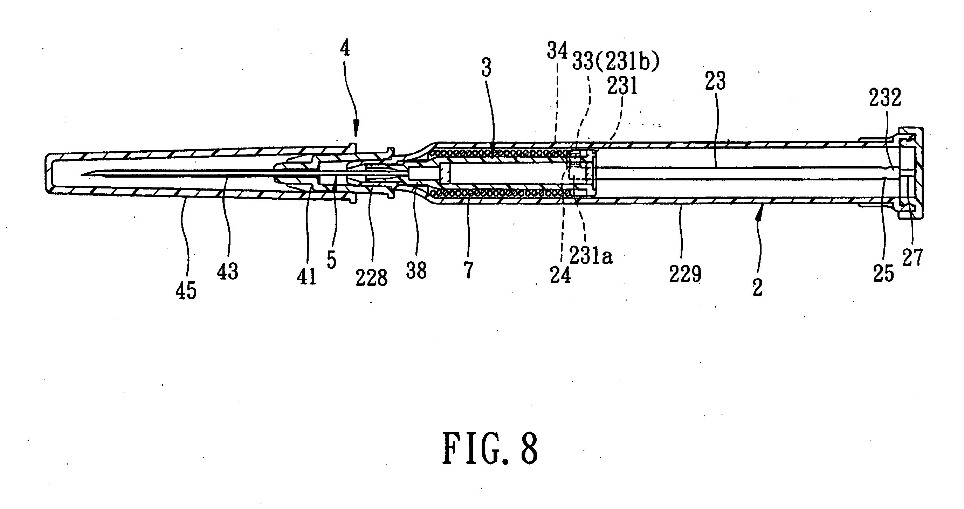

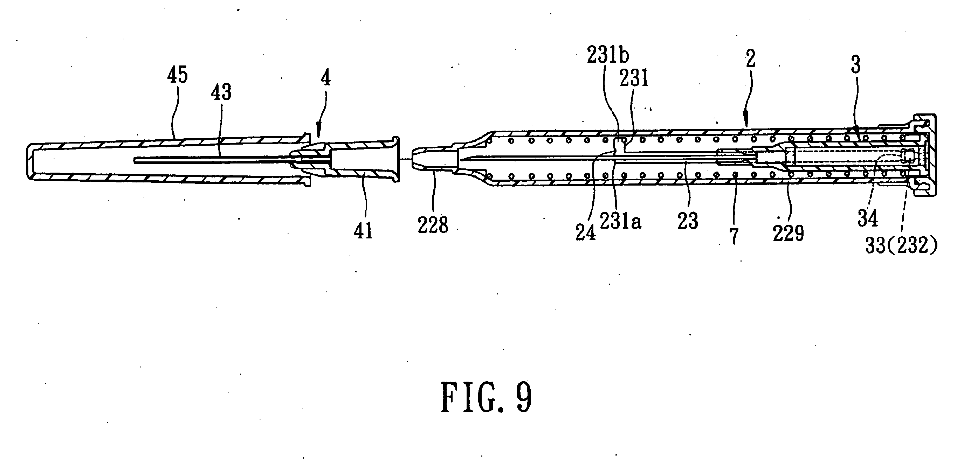

Referring to Figs. 8 and 9, the third example of an intravenous catheter introducing device is shown to be similar to the first example in construction. In this example, the retaining hole 231 includes a proximate connecting end 231a and a distal retaining end 231b which are opposite to each other in a transverse direction relative to the longitudinal direction, and which are proximate to and distal from the elongated guideway 23, respectively. The front constricted region 24 is formed between the proximate and distal connecting ends 231a, 231b. As such, the engaging peg 33 is engaged in the distal retaining end 231b to arrest the needle hub 3 at the front position. Upon retraction of the needle cannula 5, the actuator 34 is operated to move the engaging peg 33 from the distal retaining end 231b to the proximate connecting end 231a so as to permit rearward movement of the needle hub 3 along the elongated guideway 23. A coiled spring 7 is also provided to bias the needle hub 3, to the rear position as in the second preferred embodiment.

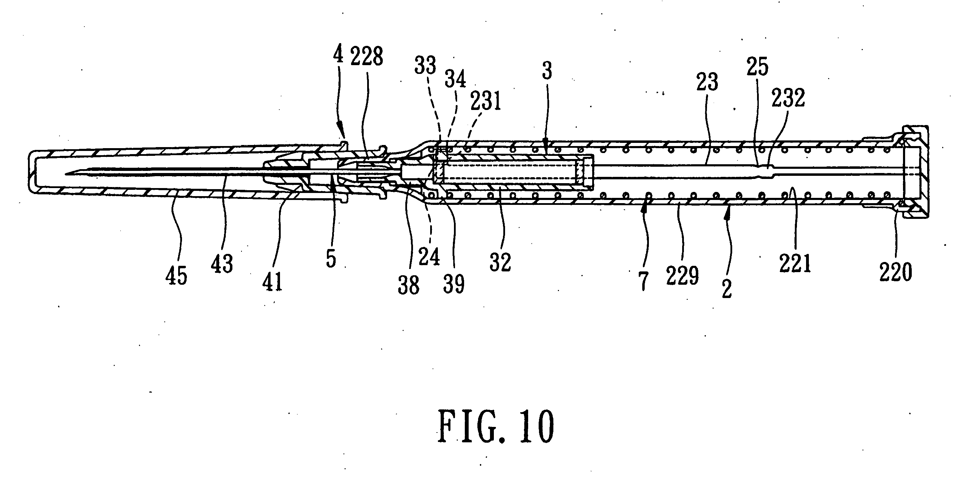

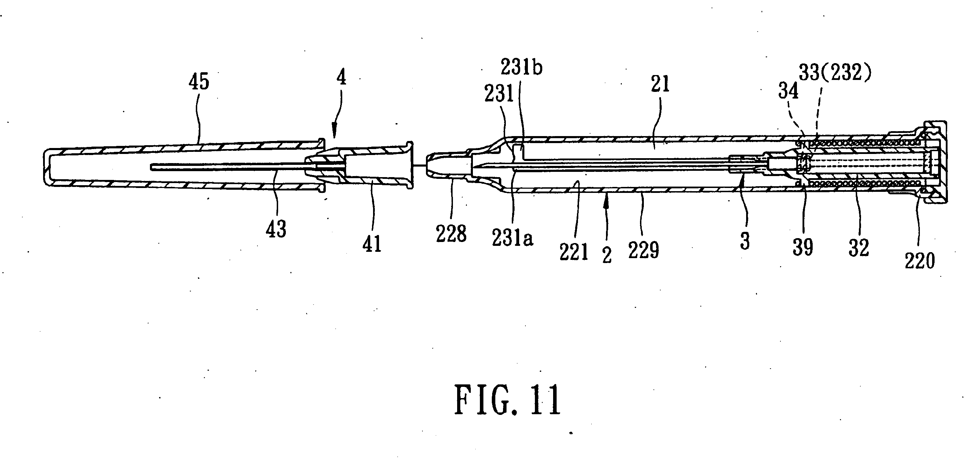

Referring to Figs. 10 and 11, the fourth example of an intravenous catheter introducing device is shown to be similar to the third example in construction. The difference resides in that the rear shell portion 32 of the needle hub 3 and the inner barrel wall surface 221 of the larger-diameter wall portion 229 respectively have an annular flange 39 and an annular rear edge 220, which are opposite to and which confront each other in the longitudinal direction so as to define a biasing member receiving space therebetween and outside of the rear shell portion 32. The coiled spring 7 is received in the biasing member receiving space, and has front and rear spring ends secured to the flange 39 and the edge 220, respectively, such that the coiled spring 7 is tensioned by the needle hub 3 when the needle hub 3 is in the front position, as shown in Fig. 10. As illustrated in the third preferred embodiment, referring to Fig 11, by operating the actuator 34 to move the engaging peg 33 from the distal retaining end 231b to the proximate connecting end 231a of the retaining hole 231, rearward movement of the needle hub 3 along the guideway 23 is permitted to enable the needle cannula 5 to be retracted into the passage 21.

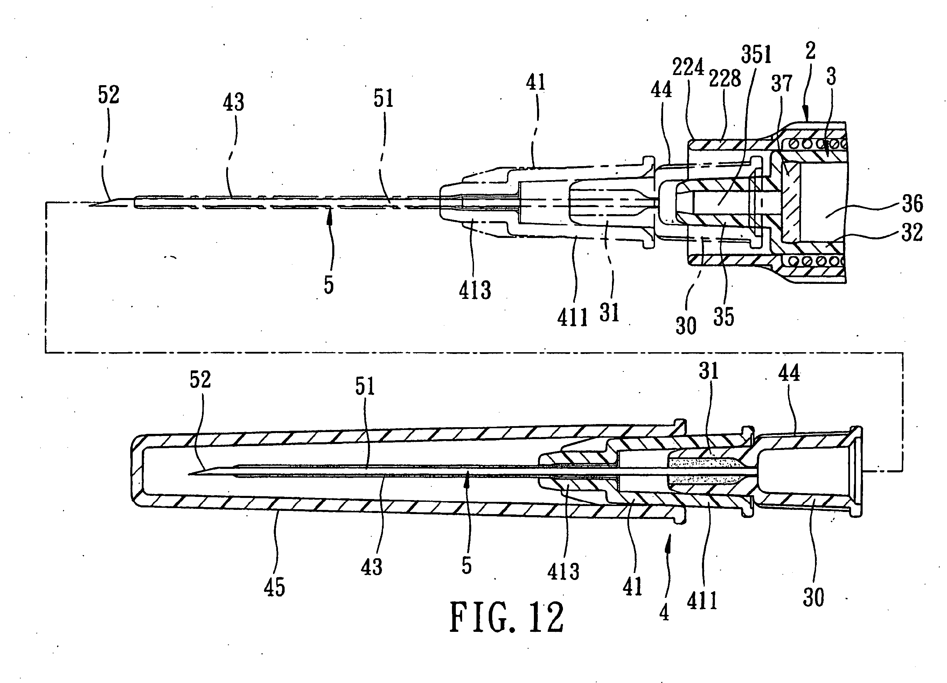

Referring to Fig. 12, the first preferred embodiment of an intravenous catheter introducing device according to this invention is shown to be similar to the previous examples in construction. The differences reside in that the smaller-diameter wall portion 228 of the barrel 2 is formed to have an increased inner diameter of the front open end 224, and that the front holding portion 31 and the rear shell portion 32 of the needle hub 3 are separated from each other. In particular, the needle hub 3 further includes an interconnecting portion 35 which is formed integrally with and which extends forwardly from the rear shell portion 32 along the axis and which defines an axial passageway 351 that extends therethrough and that is communicated with the flashback chamber 36, and a sleeve portion 30 which is integrally formed with and which extends rearwardly from the front holding portion 31 along the axis so as to form a sleeve assembly 44. The sleeve portion 30 is detachably sleeved on the interconnecting portion 35 from the front open end 224 of the barrel 2 along the axis so as to fluidly communicate the needle cannula 5 with the flashback chamber 36. Thus, the combination of the catheter connection assembly 4 and the sleeve assembly 44, which is secured with the needle cannula 5 as a functional unit, can be used as a detachable unit with different catheter dimensions for various clinical applications.

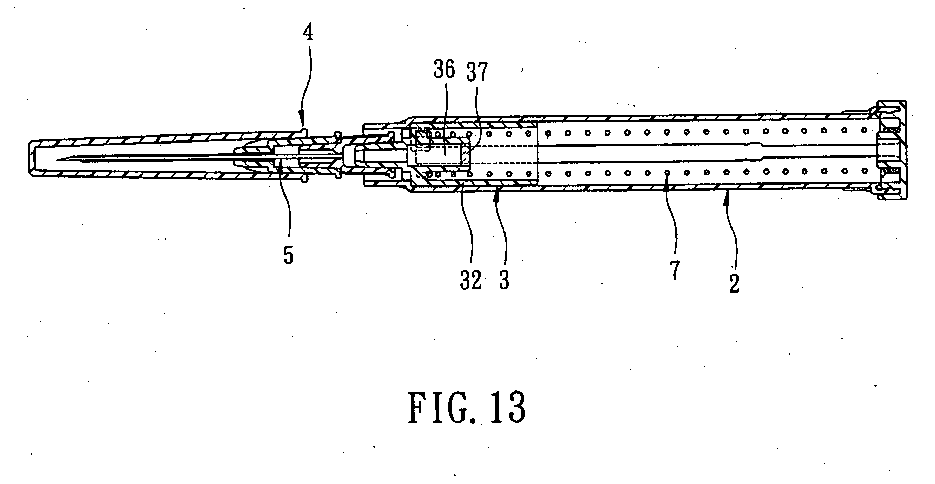

Referring to Fig. 13, the second preferred embodiment of an intravenous catheter introducing device according to this invention is shown to be similar to the first preferred embodiments in construction. The difference resides in that the coiled spring 7 has a smaller diameter, and has a front spring end received in the rear shell portion 32 of the needle hub 3 and which is secured to an inner edge of the rear shell portion 32, and a rear spring end which is secured to a closure cap 26.

## Claims

1. An intravenous catheter introducing device comprising:
a barrel (2) having front and rear open ends (224,223) opposite to each other in a longitudinal direction, and a surrounding barrel wall (22) which interconnects and which is interposed between said front and rear open ends (224,223), said surrounding barrel wall (22) including a front smaller-diameter wall portion (228) and a rear larger-diameter wall portion (229) which are opposite to each other in the longitudinal direction and which are proximate to said front and rear open ends (224,223), respectively, said surrounding barrel wall (22) having an inner barrel wall surface (221) which surrounds an axis in the longitudinal direction and which confines a passage (21) that is communicated with said front and rear open ends (224,223), and an outer barrel wall surface (222) opposite to said inner barrel wall surface (221) in radial directions relative to the axis;
a needle cannula (5) having a front segment (51) terminating at a tip end .(52), and a rear connecting end (53) opposite to said front segment (51) along the axis;
a needle hub (3) including a front holding portion (31) and a rear shell portion (32) disposed opposite to each other along the axis, said rear shell portion (32) being inserted into said passage (21) from said rear open end (223), and being slidable relative to said surrounding barrel wall (22) along the axis between front and rear positions to be proximate to said front open end (224) and said rear open end (223), respectively, said front holding portion (31) holding said rear connecting end (53) of said needle cannula (5) such that when said rear shell portion (32) is in the front position, said needle cannula (5) is placed in a position of use, where said front segment (51) extends forwardly of said front open end (224) for ready use, and when said rear shell portion (32) is in the rear position, said needle cannula (5) is placed in a disposal position, where said front segment (51) retreats into said passage (21), said rear shell portion (32) surrounding the axis and defining a flashback chamber (36) which is fluidly communicated with said needle cannula (5), wherein:
a releasably retaining member is disposed to arrest axial movement of said needle hub (3) relative to said barrel (2) when said rear shell portion (32) is in the front position, and includes
a retaining hole (231) formed in said outer barrel wall surface (222) of said larger-diameter wall portion (229), and extending in a radial direction through said inner barrel wall surface (221), and
an engaging peg (33) disposed to extend in the radial direction, and engageable in said retaining hole (231) to establish an interengagement between said larger-diameter wall portion (229) and said rear shell portion (32) such that movement of said rear shell portion (32) at the front position is arrested;
an actuator (34) operable externally and disposed to enable said engaging peg (33) to be disengaged from said retaining hole (231) so as to permit the axial movement of said needle hub (3) to the rear position;
a catheter hub (41) including a sleeve portion (411) which is detachably sleeved on said front holding portion (31) of said needle hub (3) and which defines a duct (412) along the axis, and a tip portion (413) which is opposite to said sleeve portion (411) along the axis, and which defines a through hole (414) that is communicated with said duct (412) along the axis and that permits extension of said front segment (51) therethrough; and
a tubular catheter (43) having a proximate segment (431) which is inserted into said through hole (414) and which extends along the axis to be fluidly communicated with said duct (412), and a distal segment (432) which extends from said proximate segment (431) along the axis to extend forwardly of said tip portion (413) so as to surround and sheathe said front segment (51) of said needle cannula (5) while permitting said tip end. (52) to project forwardly of said distal segment (432) when said needle cannula (5) is placed in the position of use, **characterized by**:
said front holding portion (31) and said rear shell portion (32) of said needle hub (3) being separated from each other, said needle hub (3) further including an interconnecting portion (35) which is formed integrally with and which extends forwardly from said rear shell portion (32) along the axis and which defines an axial passageway (351) that extends therethrough and that is communicated with said flashback chamber (36), and a sleeve portion (30) which is integrally formed with and which extends rearwardly from said front holding portion (31) along the axis and which is detachably sleeved on said interconnecting potion (35) from said front open end (224) of said barrel (2) along the axis so as to fluidly communicate said needle cannula (5) with said flashback chamber (36),
said sleeve portion (411) being detachably sleeved on said front holding portion (31).

## Patentansprüche

1. Vorrichtung zur Einführung eines intravenösen Katheters, die Folgendes umfasst:
einen Zylinder (2), der ein vorderes und ein hinteres offenes Ende (224, 223), die einander in einer Längsrichtung gegenüberliegen, und eine einfassende Zylinderwand (22) aufweist, die das vordere und das hintere offene Ende (224, 223) miteinander verbindet und die zwischen diesen eingeschoben ist, wobei die einfassende Zylinderwand (22) einen vorderen Wandteil mit einem kleineren Durchmesser (228) und einen hinteren Wandteil mit einem größeren Durchmesser (229) beinhaltet, die einander in der Längsrichtung gegenüberliegen und die zu dem vorderen bzw. dem hinteren offenen Ende (224, 223) benachbart sind, wobei die einfassende Zylinderwand (22) eine innere Zylinderwandfläche (221), die eine Achse in der Längsrichtung einfasst und die einen Durchlass (21) begrenzt, der mit dem vorderen und dem hinteren offenen Ende (224, 223) in Verbindung steht, und eine äußere Zylinderwandfläche (222) aufweist, die in Radialrichtungen im Verhältnis zur Achse der inneren Zylinderwandfläche (221) gegenüberliegt;
eine Nadelkanüle (5), die einen vorderen Abschnitt (51), das an einem Spitzenende (52) endet, und ein hinteres Verbindungsende (53) aufweist, das dem vorderen Abschnitt (51) entlang der Achse gegenüberliegt;
einen Nadelansatz (3), der einen vorderen Halteteil (31) und einen hinteren Hüllenteil (32) beinhaltet, die einander entlang der Achse gegenüber angeordnet sind, wobei der hintere Hüllenteil (32) von dem hinteren offenen Ende (223) in den Durchlass (21) eingeführt wird und im Verhältnis zur einfassenden Zylinderwand (22) entlang der Achse zwischen einer vorderen und einer hinteren Position verschiebbar ist, um zu dem vorderen offenen Ende (224) bzw. dem hinteren offenen Ende (223) benachbart zu sein, wobei der vordere Halteteil (31) das hintere Verbindungsende (53) der Nadelkanüle (5) derart hält, dass, wenn der hintere Hüllenteil (32) in der vorderen Position ist, die Nadelkanüle (5) in einer Gebrauchsposition positioniert ist, wobei der vordere Abschnitt (51) sich von dem vorderen offenen Ende (224) zum direkten Gebrauch nach vorne erstreckt, und wenn der hintere Hüllenteil (32) in der hinteren Position ist, die Nadelkanüle (5) in einer Abgabeposition positioniert ist, wobei der vordere Abschnitt (51) sich in den Durchlass (21) zurückzieht, wobei der hintere Hüllenteil (32) die Achse einfasst und eine Rückschlagkammer (36) definiert, die mit der Nadelkanüle (5) in Flüssigkeitsverbindung steht, wobei:
ein lösbares Rückhalteelement angeordnet ist, um Axialbewegung des Nadelansatzes (3) im Verhältnis zum Zylinder (2) zu arretieren, wenn der hintere Hüllenteil (32) in der vorderen Position ist, und Folgendes beinhaltet:
eine Rückhalteöffnung (231), die in der äußeren Zylinderwandfläche (222) des Wandteils mit einem größeren Durchmesser (229) ausgebildet ist und sich in einer Radialrichtung durch die innere Zylinderwandfläche (221) erstreckt, und
einen Eingriffsstift (33), der angeordnet ist, um sich in der Radialrichtung zu erstrecken, und in die Rückhalteöffnung (231) in Eingriff gebracht werden kann, um einen Eingriff zwischen dem Wandteil mit einem größeren Durchmesser (229) und dem hinteren Hüllenteil (32) herzustellen, so dass Bewegung des hinteren Hüllenteils (32) an der vorderen Position arretiert wird;
ein Betätigungselement (34), das von außen bedienbar ist und angeordnet ist, um zu ermöglichen, den Eingriffsstift (33) aus der Rückhalteöffnung (231) außer Eingriff zu bringen, um so die Axialbewegung des Nadelansatzes (3) zu der hinteren Position zuzulassen;
einen Katheteransatz (41), der einen Überziehmuffenteil (411), der abnehmbar über den vorderen Halteteil (31) des Nadelansatzes (3) gezogen ist und der einen Kanal (412) entlang der Achse definiert, und einen Spitzenteil (413) beinhaltet, der dem Überziehmuffenteil (411) entlang der Achse gegenüberliegt und der ein Durchgangsloch (414) definiert, das mit dem Kanal (412) entlang der Achse in Verbindung steht und das ein Erstrecken des vorderen Abschnitts (51) dort hindurch ermöglicht; und
einen röhrenförmigen Katheter (43), der einen benachbarten Abschnitt (431), der in das Durchgangsloch (414) eingeführt wird und der sich entlang der Achse erstreckt, um mit dem Kanal (412) in Flüssigkeitsverbindung zu stehen, und einen distalen Abschnitt (432) aufweist, der sich von dem benachbarten Abschnitt (431) entlang der Achse erstreckt, um sich von dem Spitzenteil (413) nach vorne zu erstrecken, um so den vorderen Abschnitt (51) der Nadelkanüle (5) einzufassen und zu überziehen, während ermöglicht wird, dass das Spitzenende (52) von dem distalen Abschnitt (432) nach vorne hervorsteht, wenn die Nadelkanüle (5) in der Gebrauchsposition positioniert ist, **dadurch gekennzeichnet, dass:**
der vordere Halteteil (31) und der hintere Hüllenteil (32) des Nadelansatzes (3) voneinander getrennt sind, wobei der Nadelansatz (3) weiterhin einen Verbindungsteil (35), der integral mit dem hinteren Hüllenteil (32) ausgebildet ist und der sich von diesem entlang der Achse nach vorne erstreckt und der einen axialen Durchgang (351) definiert, der sich dort hindurch erstreckt und der mit der Rückschlagkammer (36) in Verbindung steht, und einen Überziehmuffenteil (30) beinhaltet, der integral mit dem vorderen Halteteil (31) ausgebildet ist und der sich von diesem entlang der Achse nach hinten erstreckt und der abnehmbar von dem vorderen offenen Ende (224) des Zylinders (2) entlang der Achse über den Verbindungsteil (35) gezogen ist, um so die Nadelkanüle (5) mit der Rückschlagkammer (36) in Flüssigkeitsverbindung zu bringen,
wobei der Überziehmuffenteil (411) abnehmbar über den vorderen Halteteil (31) gezogen ist.

## Revendications

1. Dispositif d'introduction de cathéter intraveineux, comprenant :
un cylindre (2) possédant des extrémités avant et arrière ouvertes (224, 223) à l'opposé l'une de l'autre dans une direction longitudinale et une paroi de cylindre enveloppante (22) qui relie, et est intercalée entre, lesdites extrémités avant et arrière ouvertes (224, 223), ladite paroi de cylindre enveloppante (22) comportant une partie de paroi avant de plus petit diamètre (228) et une partie de paroi arrière de plus grand diamètre (229) qui se trouvent à l'opposé l'une de l'autre dans la direction longitudinale et qui sont proches desdites extrémités avant et arrière ouvertes (224, 223), respectivement, ladite paroi de cylindre enveloppante (22) ayant une surface intérieure de paroi de cylindre (221) qui entoure un axe dans la direction longitudinale et délimite un passage (21) communiquant avec lesdites extrémités avant et arrière ouvertes (224, 223), et une surface extérieure de paroi de cylindre (222) opposée à ladite surface intérieure de paroi de cylindre (221) dans des directions radiales par rapport à l'axe ;
une canule à aiguille (5) ayant un segment avant (51) qui se termine par une pointe (52) et une extrémité de raccordement arrière (53) opposée audit segment avant (51) le long de l'axe ;
un raccord d'aiguille (3) comportant une partie de maintien avant (31) et une partie d'enveloppe arrière (32) disposées à l'opposé l'une de l'autre le long de l'axe, ladite partie d'enveloppe arrière (32) étant introduite dans ledit passage (21) à partir de ladite extrémité arrière ouverte (223) et pouvant coulisser par rapport à ladite paroi de cylindre enveloppante (22) le long de l'axe entre des positions avant et arrière pour se rapprocher de ladite extrémité avant ouverte (224) et ladite extrémité arrière ouverte (223), respectivement, ladite partie de maintien avant (31) maintenant ladite extrémité de raccordement arrière (53) de ladite canule à aiguille (5) de telle façon que, lorsque ladite partie d'enveloppe arrière (32) est en position avant, ladite canule à aiguille (5) est placée dans une position d'utilisation dans laquelle ledit segment avant (51) s'étend en avant de ladite extrémité avant ouverte (224) prêt à être utilisé et lorsque ladite partie d'enveloppe arrière (32) est en position arrière, ladite canule à aiguille (5) est placée dans une position de retrait dans laquelle ledit segment avant (51) rentre dans ledit passage (21), ladite partie d'enveloppe arrière (32) entourant l'axe et définissant une chambre de retour (36) qui est en communication fluide avec ladite canule à aiguille (5),
dans lequel :
un élément de retenue déblocable qui est disposé de manière à stopper le mouvement axial dudit raccord d'aiguille (3) par rapport audit cylindre (2) lorsque ladite partie d'enveloppe arrière (32) est en position avant et qui comprend
un trou de retenue (231) formé dans ladite surface extérieure de paroi de cylindre (222) de ladite partie de paroi de plus grand diamètre (229) et s'étendant dans une direction radiale à travers ladite surface intérieure de paroi de cylindre (221), et
une cheville de blocage (33) disposée de façon à s'étendre dans la direction radiale et pouvant s'engager dans ledit trou de retenue (231) afin de mettre en prise ladite partie de paroi de plus grand diamètre (229) et ladite partie d'enveloppe arrière (32) de façon à stopper le mouvement de ladite partie d'enveloppe arrière (32) au niveau de la position avant ;
un actionneur (34) manoeuvrable de l'extérieur et disposé de façon à permettre à ladite cheville de blocage (33) de se dégager dudit trou de retenue (231) de façon à autoriser le mouvement axial dudit raccord d'aiguille (3) vers la position arrière ;
un raccord de cathéter (41) comportant une partie de manchon (411) qui est emmanchée de manière amovible dans ladite partie de maintien avant (31) dudit raccord d'aiguille (3), définissant un conduit (412) le long de l'axe et une partie de pointe (413) à l'opposé de ladite partie de manchon (411) le long de l'axe et définissant un trou traversant (414) qui communique avec ledit conduit (412) le long de l'axe et permet audit segment avant (51) de s'étendre dans celui-ci ; et
un cathéter tubulaire (43) ayant un segment proximal (431) qui est introduit dans ledit trou traversant (414) et qui s'étend le long de l'axe pour venir en communication fluide avec ledit conduit (412) et un segment distal (432) qui s'étend depuis ledit segment proximal (431) le long de l'axe de façon à s'étendre à l'avant de ladite partie de pointe (413) pour entourer et gainer ledit segment avant (51) de ladite canule à aiguille (5) tout en permettant à ladite pointe (52) de dépasser à l'avant dudit segment distal (432) lorsque ladite canule à aiguille (5) est placée dans la position d'utilisation,
**caractérisé en ce que :**
ladite partie de maintien avant (31) et ladite partie d'enveloppe arrière (32) dudit raccord d'aiguille (3) sont séparées l'une de l'autre, ledit raccord d'aiguille (3) comportant en plus une partie d'interconnexion (35) qui est formée d'un seul tenant avec, et qui s'étend à l'avant de, ladite partie d'enveloppe arrière (32) le long de l'axe et qui définit un passage axial (351) qui s'étend à travers elle et qui communique avec ladite chambre de retour (36), et une partie de manchon (30) qui est formée d'un seul tenant avec, et qui s'étend à l'arrière de, ladite partie de maintien avant (31) le long de l'axe et qui est emmanchée de manière amovible dans ladite partie d'interconnexion (35) à partir de ladite extrémité avant ouverte (224) dudit cylindre (2) le long de l'axe de façon à mettre ladite canule à aiguille (5) en communication fluide avec ladite chambre de retour (36),
ladite partie de manchon (411) étant emmanchée de manière amovible dans ladite partie de maintien avant (31).
